# EUROPEAN PATENT APPLICATION

(11) **EP 4 140 458 A1**
(43) Date of publication of application: **01.03.2023**
(21) Application number: 22191613.3
(22) Date of filing: 23.08.2022
(51) Int. Cl.: A61F 11/08

(54) **A HEARING PROTECTOR COMPRISING A HOUSING THAT IS PLACEABLE IN AN AUDITORY DUCT OF A PERSON, AS WELL AS A METHOD OF MANUFACTURING SUCH A HEARING PROTECTOR**

(30) Priority: 23.08.2021 NL 2029018
(71) Applicant: Elacin International B.V., 4817 ZL Breda (NL)
(72) Inventor: WELS, Joris, Breda (NL); FLESKENS, Bas, Breda (NL)
(74) Representative: Algemeen Octrooi- en Merkenbureau B.V.

(57) **Abstract**

A hearing protector (10) comprising a housing (30) that is placeable in an auditory duct of a person, said housing (30) having a sound damping canal (40) with a sound inlet opening (20) and a sound outlet opening (70), such that a sound passage extends in said sound damping canal (40) from said sound inlet opening (20) to said sound outlet opening (70), wherein said hearing protector (10) comprises a membrane (50) positioned in said sound damping canal (40) for providing a sound damping function, wherein said membrane (50) is integral with said housing (30), and is made of a same material as said housing (30).

## Description

The present disclosure is related to a hearing protector that is placeable in an auditory duct of a person, comprising a sound damping canal with a passage extending from a sound inlet opening to a sound outlet opening.

Such a hearing protector is known in the art and is utilized for dampening the intensity of sound to be supplied to the eardrum of a person. By varying the cross section and the length of the sound damping canal, the level of dampening can be influenced. Typically, a sound damping filter that is mountable in the hearing protector is tailored to a particular application before or after it is manufactured.

The length of the auditory duct of a person results in a resonance frequency (1/4 wavelength), which is typically between 2.5 - 3.5 kHz. Sound within this frequency range is amplified by about 10 - 15dB due to the resonant cavity effect.

Also known are filters for hearing protectors where the passage of the damping canal is fully closed off by means of a membrane. Although a hearing protector with such a filter enables a damping level that remains substantially constant in magnitude over the audible frequency range, there are disadvantages with this type of hearing protector as well.

It is found that the resonance frequency, as described above, disappears to a certain extent whenever a hearing protector is placed in the auditory duct of a person. This results in an unnatural hearing experience when a hearing protector is worn. The auditory duct is normally open at one side, and closed with the eardrum at the other side thereof. The auditory duct is no longer fully open at one side, once the hearing protector with membrane is placed therein.

One of the downsides of the known hearing protectors is that they are not sufficiently customizable. That is, the manufacturing process is often bound to a limited number of predetermined pre-sets. Additionally, the manufacturing process of known hearing protectors is relatively cumbersome.

It is therefore an object of the present disclosure to provide for a hearing protector that can be manufactured in a fully customizable and cost-effective manner.

It is a further object to provide for a method of manufacturing such a hearing protector.

In a first aspect, there is provided a hearing protector comprising a housing that is placeable in an auditory duct of a person, said housing having a sound damping canal with a sound inlet opening and a sound outlet opening, such that a sound passage extends in said sound damping canal from said sound inlet opening to said sound outlet opening, wherein said hearing protector comprises a membrane positioned in said sound damping canal for providing a sound damping function, wherein said membrane is integral with said housing, and is made of a same material as said housing.

The difference with the prior art hearing protector is that the membrane of the hearing protector is formed integrally with the housing. There is no separate filter as a second component that is placed inside the hearing protector. The hearing protector in accordance with the present disclosure consists of one single component, that is e.g. made in a single, continuous process step.

The housing is construed in such a way that the damping canal is defined by the housing. The damping canal may have a sound inlet opening that is larger than the sound outlet opening. This may improve the sound dampening process.

The design as a single component advantageously allows for more design freedom and customization as, compared to previously-known designs, there is no longer a need for inserting a separate filter into the hearing protector. It is noted that the dimensions of the sound damping canal may be developed such that the damping is tuned to a particular frequency range, for example 2.5 - 3.5 kHz. Adequate tuning of the dimensions of the housing, and thereby the sound damping canal, allows for a tailored acoustic resistance which can replace any present resonance over the frequency range. Designing the hearing protector as a single component makes available more options to a developer designing the hearing protector of an individual user, as the developer may use all aspects of the housing design as a variable for creating the sound damping canal instead of just the plugin filter as is nowadays the practice.

In an example, the housing and membrane are integrally 3D printed.

The above means that the hearing protector may be printed, as a whole, using a single type of material. The hearing protector may, for example, be printed layer by layer thus including printing of the membrane itself.

Typical layer thicknesses may include 100 µm or anything alike.

In prior art solutions, typically, a mold of a rigid and hard material was 3D printed. The mold was used to receive material for creating the housing of the hearing protector. Only after the material forming the housing was solidified, the mold was destroyed such that a hearing protector was obtained.

The present example aims at directly printing the hearing protector itself, as a single piece. Advantageously, there is no need to work with a mold anymore.

In a further example, the membrane is irremovably attached to said housing.

As mentioned above, the membrane is created along with the housing, i.e. in one go. This means that the membrane is no longer removable and replaceable in the housing. As such, the membrane is irremovable attached to the housing.

In a further example, the material is a soft silicon material.

The soft silicon material may be, for example, based on an additive manufacturing composition comprising: at least one alkenylderivatized polysiloxane and at least one mercapto-derivatized polysiloxane crosslinking agent of formula:

R¹(R)₂SiO((R³R⁴)SiO)ₓSi(R)₂R²,

wherein R is independently C(1-12)alkyl, preferably methyl;
R¹, R², R³, R⁴, are independently C(1-12)alkyl, preferably methyl, or mercapto-C(1-12)alkyl;
X is 1 or more, preferably 1 to 100;
with the provisio that at least one of R¹, R², R³, ^{R}₄ is mercapto-C(1-12)alkyl, and wherein the alkenyl-derivatized polysiloxane and the mercapto-derivatized polysiloxane crosslinking agent form a stable emulsion.

In a further example, the hearing protector comprises at least two membranes that are each integral with said housing, and are each of the same material as said housing.

The advantage of using two membranes is that more dampening may be provided and/or the dampening may be better tailored to the specific required frequencies.

Different types of membranes may be created in this way that each accomplish a particular damping characteristic. For example, the membrane may be impermeable to air, or may on the contrary be permeable to air. The membrane may have a closed surface, or may comprise one or more holes or micropores, in basically any position / pattern / size imaginable. By increasing the number of openings and/or the diameter thereof, the damping of low frequencies by the membrane can be reduced. By providing perforations of substantially equal diameter, the low frequency damping can simply and reproducibly be influenced in a stepwise manner. These characteristics of the membrane are in principle independent of the material of which the membrane is made.

In another example, the housing is formed such that:
- said sound inlet opening has a diameter of between 4mm - 6mm, preferably substantially 5mm.

In a further example, the housing is formed such that:
- said sound outlet opening has a diameter of between 0,3mm - 1,0mm, preferably substantially 0,6mm.

It was found that the above described ranges for the sound inlet opening and the sound outlet opening are beneficial for the damping characteristic of the hearing protector.

Typically, it is desired to obtain a relatively flat damping characteristic over the frequency. The damping should, ideally, be completely flat over the full frequency range, except for in the resonance frequency range. In the resonance frequency range, the damping is to be reduced to compensate for the missing resonance frequency due to the placement of the hearing protector. This can be obtained with a sound outlet that is smaller than a sound inlet.

In a second aspect of the present disclosure, there is provided a method of manufacturing a hearing protector in accordance with any of the previous examples, wherein the method comprises the step of:
- integrally construing the housing of the hearing protector and the membrane of the hearing protector, using the same material.

It is noted that the advantages as explained with respect to the first aspect, being the hearing protector, are also applicable to the second aspect, being a method of manufacturing the hearing protector.

In an example, the step of integrally construing comprises 3D printing said housing and said membrane using said material.

In a further example, the step of integrally construing comprises irremovably construing said membrane to said housing.

In an example, the material is a soft silicon material.

In another example, the step of integrally construing comprises integrally construing at least two membranes that are integral with said housing, using the same material.

In yet a further example, the step of integrally construing comprises integrally construing said housing such that said sound inlet opening has a diameter of between 4mm - 6mm, preferably substantially 5mm.

In another example, the step of integrally construing comprises construing said housing such that said sound outlet opening has a diameter of between 0,3mm - 1,0mm, preferably substantially 0,6mm.

In yet other examples, the membrane is impermeable to air or the membrane is permeable to air. When the membrane is permeable to air, it may e.g. comprise one or more openings.

These and other aspects of the invention will be apparent from and elucidated with reference to the embodiment(s) described hereinafter.

### Brief description of the Drawings

Figure 1 discloses an open-worked hearing protector in accordance with the present disclosure;
Figure 2 discloses a cross-sectional view of the hearing protector of Figure 1; and
Figure 3 discloses a hearing protector is in accordance with the prior art.

### Detailed description

It is noted that the figures are only schematic representations of preferred embodiments of the disclosure.

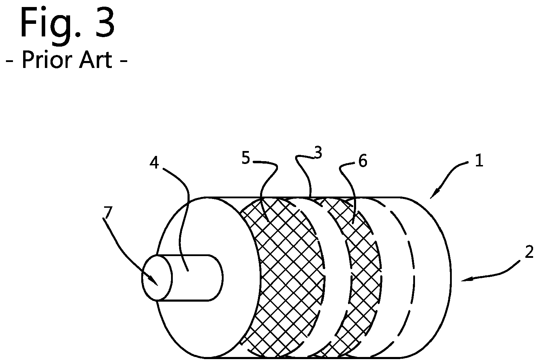

To start with Figure 3, shown is an example of a filter element 1 of a hearing protector in accordance with the prior art. Such a filter element 1 of the prior art is typically injection moulded. The a hearing protector can be placed in the auditory duct of a person. The hearing protector comprises a housing surrounding the outside 3 of the filter element 1, the housing defining a sound damping canal. Arranged in the sound damping canal of the housing is the filter element 1. The filter element 1 in turn comprises two membranes 5, 6. The canal 4 extending through the filter element 1 comprises three tube parts, a first tube part in between the inlet 2 of the canal 4 and the first membrane 6, a second tube part in between the second membrane 5 and the outlet 7 of the canal 4, and a third tube part in between the two membranes 5, 6. The lengths and diameters of the respective tube parts can mutually vary in the prior art hearing protector.

The prior art hearing protector is manufactured by making a mould that corresponds to a shape of the wearers auditory duct. The mould is filled with a molten material, e.g. a silicon material, to make the housing. Once the material is hardened, the mould is destroyed and the housing of hearing protector is obtained. After making housing, the sound damping canal is created therein, following which the filter element 1, which is a separate element, has to be inserted into the sound damping canal. This is relatively cumbersome as the dimensions are small. Also, only a select number of filters 1 can realistically be kept in stock by a hearing protector manufacturer, such that the optimal design of a hearing protector for a wearer, based on tests performed for said wearer, is always a trade-off between the optimal filters 1 in a theoretical solution, and the solution that can be achieved with the filters 1 kept in stock by the manufacturer.

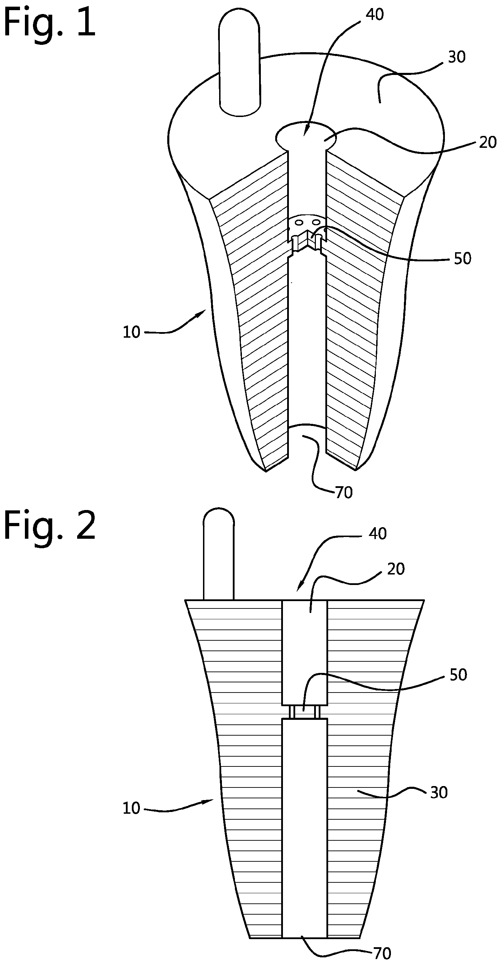

Turning now to Figures 1 and 2, which will be described simultaneously, a hearing protector 10 according to the present invention is shown. The hearing protector 10 has a housing 30 that is placeable in an auditory duct of a person. The housing 30 has a sound damping canal 40 with a sound inlet opening 20 and a sound outlet opening 70, such that a sound passage extends in the sound damping canal 40 in between the inlet opening 20 and the outlet opening 70. In the embodiment shown here, the sound inlet opening 20 of the hearing protector 10 has the same diameter as the sound outlet opening 70, however these openings 20, 70 may also have different sizes. For example, the sound inlet opening 20 may be larger than the sound outlet opening 70, or the sound outlet opening 70 may be larger than the sound inlet opening 20. As shown here, the canal 40 has a substantially straight shape, with a diameter of the canal 40 being constant over its entire length. However, the shape of the canal may also be curved, slanted, tapered, conical, or have yet another shape. The diameter may also vary, e.g. continuously or via one or more step changes.

The hearing protector 10 further comprises a membrane 50 positioned in the sound damping canal 40 for providing a sound damping function. The membrane 50 is positioned between the sound inlet opening 20 and the sound outlet opening 70, such that the sound dampening canal 40 is comprised of equal or non-equal lengths. The present figures shows the membrane 50 is positioned closer to the inlet opening 20, than to the outlet opening 40, thus having a sound dampening canal 40 comprising two non-equal lengths. A skilled person in the art will understand that alternatively, the position of the membrane 50 in the dampening canal 40 may be positioned closer towards either sound inlet 20 or sound outlet 40. Positioning the membrane 50 at a specific position within the sound dampening canal 40 allows the modulation of the sound dampening properties of the hearing protector 10. By varying the position of the membrane 50 within the sound dampening canal 40, the level of dampening can be influenced. The present figures shows one membrane 50 but one skilled in the art will understand that alternatively there may be two, three or even more membranes 50. Advantageously, as shown, the membrane 50 is formed integrally with the housing 30, and is made of the same material as the housing 30. As such, the hearing protector 10 is made as a single component, without the need for any assembly of different components before the product can be send to a user. This reduces production time, makes production less complicated with less steps, less room for error and, in embodiments, more design freedom.

For example, the housing 30 and the membrane 50 can be 3D printed. In embodiments this allows a maximum amount of freedom in the design of the hearing protector 10, as the 3D print can be a unique print for each user, optimally tailoring the hearing protector 10 to the specific sound damping needs of the user. For example, when 3D printing the hearing protector 10, not only the outer shape of the housing 30 can be optimally shaped to fit into the auditory duct of the wearer, but also the membrane(s) 50 can be optimally designed to fit the specific needs of the wearer, and even the size, including length and diameter, and shape of the sound damping canal parts can be tailored to the specific needs of a person.

For example, the number, position, pattern and diameter of openings in the membrane 50 can be varied and set at specifications best suiting the needs of the individual wearer when 3D printing the housing 30 and membrane 50. The number of openings may include zero, such that the membrane 50 has a closed surface, e.g. being impermeable to air. Also, the thicknesses of the membrane 50 can easily be varied when 3D printing the hearing protector 10. All these factors play a role in obtaining the "perfect" hearing protector for an individual having certain sound damping requirements / needs. All these factors can now be customized with great ease to obtain the most natural sound experience while wearing hearing protectors.

A consequence of forming the membrane 50 integral with the housing 30, is that the membrane 50 may be irremovably attached to the housing 30, the housing 30 and membrane 50 forming a single component that cannot be taken apart.

The present disclosure further relates to a method of manufacturing a hearing protector 10 as described in the above, wherein the method comprises at least the step of integrally construing the housing 30 of the hearing protector 10 and the membrane 50 of the hearing protector 10, while using the same material.

It is noted that the present disclosure is not limited to just the embodiments as disclosed in the above, but that the teaching as presented herein can be modified and enhanced by those skilled in the art beyond the scope of the present disclosure, as defined in the appended claims, without having to apply inventive skills.

## Claims

1. A hearing protector (10) comprising a housing (30) that is placeable in an auditory duct of a person, said housing (30) having a sound damping canal (40) with a sound inlet opening (20) and a sound outlet opening (70), such that a sound passage extends in said sound damping canal (40) from said sound inlet opening (20) to said sound outlet opening (70), wherein said hearing protector (10) comprises a membrane (50) positioned in said sound damping canal (40) for providing a sound damping function, wherein said membrane (50) is integral with said housing (30), and is made of a same material as said housing (30).

2. The hearing protector in accordance with claim 1, wherein said membrane (50) is positioned between the sound inlet opening (20) and the sound outlet opening (70), such that the sound dampening canal (40) is comprised of equal or non-equal lengths.

3. The hearing protector in accordance with any of the previous claims, wherein said housing (30) and membrane (50) are integrally 3D printed.

4. The hearing protector in accordance with any of the previous claims, wherein said membrane (50) is irremovably attached to said housing (30).

5. The hearing protector in accordance with any of the previous claims, wherein said material is a soft silicon material.

6. The hearing protector in accordance with any of the previous claims, wherein said hearing protector (10) comprises at least two membranes (50) that are each integral with said housing (30), and are each made of the same material as said housing (30).

7. The hearing protector in accordance with any of the previous claims, wherein the membrane (50) is permeable to air, the membrane (50) comprising one or more cavities.

8. The hearing protector in accordance with any of the previous claims, wherein said membrane is impermeable to air and comprises a closed membrane surface.

9. A method of manufacturing a hearing protector (10) in accordance with any of the previous claims, wherein said method comprises the step of:
- integrally construing the housing (30) of the hearing protector (10) and the membrane (50) of the hearing protector (10), using the same material.

10. The method in accordance with claim 8, wherein said step of integrally construing comprises 3D printing said housing (30) and said membrane (50) using said material.

11. The method in accordance with claim 8 or 9, wherein said step of integrally construing comprises irremovably construing said membrane (50) to said housing (30).

12. The method in accordance with any of the claims 8 - 10, wherein said material is a soft silicon material.

13. The method in accordance with any of the claims 8 - 11, wherein said step of integrally construing comprises construing at least two membranes (50) that are integral with said housing (30), using the same material.

14. The method in accordance with any of the claims 8 - 12, wherein said step of integrally construing comprises construing a membrane (50) having one or more cavities.

15. The method in accordance with any of the claims 8 - 13, wherein said step of integrally construing comprises construing a membrane including a closed membrane surface.
